# EUROPEAN PATENT SPECIFICATION

(11) **EP 3 335 711 B1**
(45) Date of publication and mention of the grant of the patent: **09.12.2026**
(21) Application number: 17208377.6
(22) Date of filing: 30.03.2011
(51) Int. Cl.: A61K 31/415, A01N 43/52

(54) **FORMULATIONS OF MAZINDOL**
FORMULIERUNGEN AUS MAZINDOL
FORMULATIONS DE MAZINDOL

(30) Priority: 31.03.2010 US 282788 P
(43) Date of publication of application: 20.06.2018
(62) Document number(s) of the earlier application(s) in accordance with Art. 76 EPC: 11763348.7 / 2 552 210
(73) Proprietor: Supernus Pharmaceuticals, Inc., Rockville, MD 20850 (US)
(72) Inventor: KIDANE, Argaw, Montgomery Village, MD 20886 (US); BHATT, Padmanabh, Rockville, MD 20850 (US)
(74) Representative: Louis Pöhlau Lohrentz

(56) References cited:
- WO-A1-2009/155139
- KR-A- 20070 081 903
- US-A- 3 597 445
- US-A1- 2006 240 105
- US-A1- 2009 136 593
- US-A1- 2009 317 471
- US-A1- 2010 160 363
- ANONYMOUS: "Lactose - Some basic properties and characteristics", WWW.DFEPHARMA.COM, 28 October 2011 (2011-10-28), http://www.dfepharma.com, pages 1 - 12, XP055209278, Retrieved from the Internet <URL:https://web.archive.org/web/20110801000000*/http://www.dfepharma.com/en/downloads.aspx?id={2BB38A30-33EC-488D-9B1F-FEE0AD797636}> [retrieved on 20150824]

## Description

### CROSS-REFERENCE TO RELATED APPLICATIONS

This application claims priority to US provisional application no. 61/282,788 filed on March 31, 2010.

### BACKGROUND OF THE INVENTION

Mazindol, (RS)-5-(4-chlorophenyl)-3,5-dihydro-2H-imidazo[2,1-a]isoindol-5-ol, is a sympathomimetic amine, which is similar to amphetamines. It is also known as an "anorectic" or an "anorexigenic" drug. Mazindol stimulates the central nervous system which increases heart rate and blood pressure and decreases appetite.

Mazindol exhibits instability at alkaline pH condition, especially in the presence of water. Thus, development of a stable immediate or modified release formulation of mazindol presents a challenge that is solved by the instant invention. KR-10-2007-0081903 is directed to a pharmaceutical composition containing mazindol having enhanced stability.

### SUMMARY OF THE INVENTION

In one embodiment, the current invention is directed towards stable immediate release (IR) and modified release formulations of mazindol according to claim 1 that comprise not more than 5% by weight of the formulation of water. Further, the modified release formulations optionally comprising stabilizing agents are also disclosed. The modified release formulation is a delayed release (DR) formulation. The modified release formulation can be a formulation that provides a pulsatile release. The pulsatile release may be achieved using a combination of an extended release with a delayed release, or immediate release with an extended release, or immediate release with a delayed release, or immediate release with an extended release and delayed release.

Stable immediate release formulations of mazindol that comprise not more than 5% of water by weight of the formulation can be provided..

The further embodiment covers a dosage form containing the formulation of the current invention wherein said dosage form is selected from capsules. The capsules may contain pellets, beads, tablets, mini-tablets, granules, and/or powders. Capsules may also be soft gelatin capsules containing non-aqueous or partially non-aqueous fill. The formulation may be also presented in the form of pellets in a capsule, where the capsule can be opened and the pellets sprinkled on to soft food or in a liquid and then swallowed.

Although many of the embodiments and discussion herein are with respect to mazindol per se, the invention should not be so limited. The present invention also contemplates the hydrolysis product of mazindol (HP, chemical name: 2-(2-Aminoethyl)-3-(4-chlorophenyl)-3-hydro-2,3-dihydroxy-1H-isoindol-1-one), and/or prodrugs of mazindol and/or prodrugs of the hydrolysis product of mazindol for administration to mammals to treat CNS disorders.

Further, the present invention provides a once-a-day dosage form of mazindol and/or hydrolysis product thereof and/or prodrug thereof and/or salt thereof delivering to a mammal a therapeutically effective amount of the active ingredient for the treatment of CNS disorders, including but not limited to the treatment of ADHD.

Additionally, stabilized formulations of mazindol prepared from mazindol starting material having low level of impurities are also disclosed.

In an additional embodiment, the invention also provides a dosage form of mazindol that can provide therapeutic levels of the drug for the period of time from 6 to 24 hours.

### BRIEF DESCRIPTION OF THE DRAWINGS

Figure 1 shows the dissolution profile for IR pellets of Example 1.
Figure 2 shows the dissolution profile for DR1 pellets of Example 2.
Figure 3 shows the dissolution profile for IR/DR1 Capsules, 1.5mg, using USP Apparatus II at 50RPM and media of 0.1N HCl (pH 1.1) for the first 2 Hrs followed by media adjustment to pH 6.8 using 50mM phosphate buffer (Example 3).
Figure 4 shows the dissolution profiles of the IR pellets using USP Apparatus II at 50RPM and 0.1N HCl (pH 1.1) dissolution media (Example 4).
Figure 5 shows the dissolution profiles of the IR pellets using USP Apparatus II at 50RPM and 0.1N HCl (pH 1.1) dissolution media (Example 5)
Figures 6 and 7 show the dissolution profiles for mazindol IR tablets (Examples 7 and 8).
Figure 8 shows the dissolution profile for mazindol DR tablets (Example 9).
Figure 9 shows the dissolution profiles for the mazindol IR tablets containing anhydrous lactose (Example 10).
Figure 10 shows the dissolution profiles of the AMG seal coated IR tablets (Example 11).
Figure 11 shows the dissolution profile for the DR tablets (Example 12).
Figure 12 shows stability profiles of mazindol IR and DR tablets in various formulations (Example 14).
Figure 13 shows the effect of Aquarius Moisture Guard (AMG) coating on the stability of mazindol IR and DR formulations (Example 14).
Figure 14 shows the dissolution profiles for mazindol extended release tablets (Example 13).
Figure 15 shows *in-silico* generated dissolution profiles with varying lag times (Example 15).
Figure 16 shows *in-silico* generated pharmacokinetic profiles (Example 15).
Figure 17 shows the level of impurities in mazindol drug substance before and after the washing step.
Figure 18 shows the stability profiles of Mazindol IR Capsules, 1.5mg, in terms of the growth of the hydrolysis product of mazindol.
Figure 19 shows the stability profile of Mazindol IR Capsules, 1.5mg, in terms of the growth of the total non-parent peak (NPP).
Figure 20 shows the dissolution profiles of the mazindol IR prototypes tested in dogs (Example 19).
Figure 21 shows the pharmacokinetic profiles of mazindol in dogs dosed with mazindol tablet and capsule prototypes (Example 19).
Figure 22 shows the pharmacokinetic profiles of the hydrolysis product of mazindol (HP) in dogs dosed with mazindol tablet and capsule prototypes (Example 19).

### DEFINITIONS

Unless otherwise specified, "a" or "an" means "one or more" in the present application.

The term "mazindol" means (RS)-5-(4-chlorophenyl)-3,5-dihydro-2H-imidazo[2,1-a]isoindol-5-ol or a pharmaceutically acceptable salt or ester thereof, as well as variable mixtures of the R and S enantiomers or either one of the R or S enantiomers in a substantially pure form.

An "immediate release formulation" refers to a formulation that releases greater than or equal to about 80% by weight of the active pharmaceutical agent in less than or equal to about 1 hour.

The term "modified release" encompasses any mode of release that is different from the immediate release.

In the current application, the term "non-pH dependent polymers" is used to mean "polymers having solubility that is not pH-dependent" and the term "pH dependent polymers" is used to mean "polymers having solubility that is pH-dependent";

For the purposes of this application, terms "pH-dependent polymers" and "enteric polymers" are used interchangeably.

The term "particles", as used herein, includes, without any limitations on the nature and size thereof, any particles, spheres, beads, granules, pellets, particulates or any structural units that may be incorporated into an oral dosage form.

The term "impurity" refers throughout this application to any entity different from the active ingredient(s), water or excipients . For example, HP may be considered an impurity where mazindol is the intended active ingredient.

### DETAILED DESCRIPTION OF THE INVENTION

The references to methods of treatment in the subsequent paragraphs of this description are to be interpreted as references to the compounds, pharmaceutical compositions and medicaments of the present invention for use in a method for treatment of the human or animal body by therapy or for diagnosis. Though mazindol drug substance is chemically stable, its stability in the immediate or modified release formulations is often compromised because it appears that mazindol is not compatible with many commonly used pharmaceutical excipients. A combination of mazindol with these excipients results in significant degradation of the active agent.

It was unexpectedly discovered that the problem of instability may be solved by keeping the total amount of water in the formulation to a very low level, less than 5%, preferably less than 2%, by weight of the formulation.

It was also discovered, surprisingly, that stability of a mazindol formulation can be improved by using the mazindol drug starting material with substantially reduced level of impurities. Thus the current invention provides for mazindol, the starting material, having a total amount of impurities (e.g., HP) less than 1.0% of the active ingredient, preferably less than 0.5%, more preferably less than 0.25%, and most preferably, less than 0.1%. Accordingly, the current invention provides for formulations of mazindol wherein the total amount of impurities (e.g., HP) is less than 5% of the active ingredient, preferably less than 2.5%.

It was further discovered that stable formulations of mazindol may be prepared with the use of certain excipients (referred to herein as "stabilizing excipients"). In one embodiment of the invention, excipients are acidifiers selected from the group consisting of fumaric acid, citric acid, malic acid, tartaric acid, ascorbic acid, edetic acid, aspartic acid, adipic acid, alginic acid, benzoic acid, butandioic acid, erythorbic acid, lactic acid, malic acid, maleic acid , glutamic acid, sorbic acid, succinic acid, acacia, aluminum phosphate, aluminum sulfate, ammonium alum, ammonium chloride, carbomers, edetate calcium disodium, edetate disodium, methacrylic acid copolymers, polycarbophils, polydextrose, potassium alum, potassium phosphate monobasic, sodium metabisulfite, sodium phosphate monobasic, sodium starch glycolate, zinc acetate and zinc sulfate, pharmaceutical grade ion exchange resins such as Amberlite IRP64, Amberlite IRP68, Amberlite IRP69, Amberlite IR120, Dowex 50, and combinations thereof.

In another embodiment of the invention, stabilizing excipients are selected from hydrophobicity inducing (hydrophobizing) agents. These agents may be represented by magnesium stearate, stearic acid, glyceryl behenate, glyceryl stearate, glyceryl palmitostearate, waxes and hydrogenated vegetable oils, among others known to those of ordinary skill in the art. Combinations of these excipients may also be used.

Stabilizers may be incorporated into the formulations of mazindol in a variety of ways. They may be intermixed with the active ingredient and/or other excipients, or may be provided in the form of a coating on the mazindol-containing substrate. Alternatively, excipients, such as bulking agents, may be pre-treated by the stabilizers prior to their incorporation into the formulation. Stabilization of mazindol may be also achieved by coating drug loaded core substrates such as pellets and tablets with coating polymers dissolved or dispersed in acidic solution.

These and further ways of using stabilizers are disclosed in more details in the examples below.

Additional excipients that can be used to formulate stable mazindol drug products in accordance with the current invention include bulking agents, such as lactose anhydrous, lactose monohydrate, Supertab 21AN, Ludipress, Ludipress LCE, Fast Flo Lactose, Supertose, Pharmatose, Respitose, glyceryl behenate, and hypromellose; wetting and solubility enhancing agents, such as sodium lauryl sulfate, polyethylene glycol, PEG glyceryl esters, lecithin, poloxamer, the polysorbates, the polyoxyethylene alkyl ethers, polyethylene castor oil derivatives, polysorbates, polyethylene stearate, and the sorbitan esters; fillers such as low moisture microcrystalline cellulose (Avicel^{®} grades PH-103, PH-112, PH-113, PH-200), colloidal silicon dioxide, dextrates (anhydrous), dextrose (anhydrous), maltol, fructose, glyceryl palmitostearate, glyceryl monostearate, guar gum, lactitol anhydrous), lactose (anhydrous), lecithin, magnesium carbonate, maltitol, maltose, mannitol, poloxamer, polyethylene oxide, sorbitol, sucrose, compressible sugar, confectioner's sugar, xylitol. These excipients may be used separately or in combinations.

Through use of stabilizers and excipients with low levels of moisture as described above, the inventors were able to realize one goal of the current invention: to provide stable immediate release formulations of mazindol that comprise not more than 5% of water by weight of the formulation. In yet further embodiment, the invention discloses stable immediate release formulations of mazindol comprising stabilizing excipients.

A further goal of the current invention is to utilize stabilization techniques described above to provide stable modified release formulations of mazindol comprising mazindol, at least one release controlling polymer that may be a non-pH-dependent polymer or a pH-dependent, enteric polymer, or a combination thereof, and at least one pharmaceutically acceptable excipient. Further, the invention provides modified release formulations of mazindol comprising mazindol, at least one release controlling polymer and one or more pharmaceutically acceptable excipients selected from those described above, wherein the total amount of residual water in the formulation is not more than 5% by weight of the formulation.

Further, the invention provides modified release formulations of mazindol where the total amount of impurities in mazindol drug substance does not exceed 2.5%; preferably does not exceed 2%; and even more preferably does not exceed 1%. A synergistic enhancement of stability is achieved by employing high-purity mazindol and low - moisture excipients, or low-moisture excipients and acidic excipients, or high-purity mazindol and low-moisture excipients and acidic excipients, as discussed above.

The modified release formulations of mazindol exhibiting extended release profile, or delayed release profile, or combination of extended release and delayed release profile, or any combination of those with an immediate release profile are disclosed herein. In some embodiments, the formulations may exhibit a pulsatile release profile. These specific release profiles are achieved by formulating mazindol, at least one release controlling polymer and at least one excipient in a variety of inventive formulations.

The release controlling polymers of the current invention may be selected from non-pH-dependent polymers such as hydrophilic rate controlling compounds that can be used to formulate modified release multiparticulates or matrix tablets drug products, and hydrophobic rate controlling compounds that exhibit limited or no water solubility; or enteric polymers that exhibit pH-dependent solubility. The following non-limiting examples of such compounds are provided below:

Hydrophilic compounds: hydroxypropyl cellulose, hypromellose (hydroxypropyl methyl cellulose), methyl cellulose, polyethylene oxide, acacia, acrylic acid derivatives (e.g., carbomer homopolymer type A NF and carbomer homopolymer type B NF), hydroxyethyl cellulose, carrageenan, tragacanth, xanthan gum, povidone, alginic acid (and salts thereof), polyvinyl alcohol, carboxymethylcellulose, and combinations thereof.

Hydrophobic compounds: ethylcellulose, cellulose acetate, cellulose acetate butyrate, waxes (e.g., carnauba wax, microcrystalline wax), hydrogenated vegetable oils, Compritol 888 ATO (glyceryl behenate), Precirol ATO 5 (glyceryl palmitostearate), PEG glyceryl esters such as Gelucire 50/1, Eudragit^{®} NE30D or Eudragit NM30D poly(ethyl acrylate-co-methyl methacrylate) ethyl acrylate methyl methacrylate copolymer, Eudragit^{®} RS and Eudragit^{®} RL poly (ethyl acrylate-co-methyl methacrylate-cotrimethylammonioethyl methacrylate chloride), polyvinyl acetate, cellulose acetate propionate, and combinations thereof.

pH-dependent compounds: Eudragit^{®} FS30D (poly (methyl acrylate-co-methyl methacrylate-co-methacrylic acid)), Eudragit^{®} L30D-55, Eudragit^{®} L and Eudragit^{®} S (poly (methacrylic acid-co-methyl methacrylate)), hydroxypropyl methylcellulose acetate succinate, hydroxypropyl methylcellulose phthalate, cellulose acetate phthalate, shellac, zein, and combinations thereof.

The release controlling polymer (non- pH-dependent polymer, pH-dependent polymer or combination of both) may be included into the formulation in the amount of from 5% to 99%, preferably in the amount of from 5% to 75%, most preferably in the amount of from 5% to 50%, by weight of the formulation.

Non-pH-dependent polymers that can be used for coating multiparticulates or tablets (matrix or immediate release) include: cellulose esters, cellulose acetate, cellulose acetate butyrate, ethylcellulose, Eudragit^{®} RS and Eudragit^{®} RL poly (ethyl acrylate-co-methyl methacrylate-cotrimethylammonioethyl methacrylate chloride), Eudragit^{®} NE30D or Eudragit NM30D poly(ethyl acrylate-co-methyl methacrylate), ethyl acrylate methyl methacrylate copolymer, polyvinyl acetate and combinations thereof.

In addition, the following enteric compounds can be used in a coating to provide a delay in the release profile: Eudragit^{®} FS30D (poly (methyl acrylate-co-methyl methacrylate-co-methacrylic acid)), Eudragit^{®} L30D-55, Eudragit^{®} L and Eudragit^{®} S (poly (methacrylic acid-co-methyl methacrylate)), hydroxypropyl methylcellulose acetate succinate, hydroxypropyl methylcellulose phthalate, cellulose acetate phthalate, shellac, zein, and combinations thereof.

These polymers may be used to prepare a variety of modified release systems:
A) Matrix systems, wherein an active pharmaceutical ingredient (mazindol, or mazindol and an additional active); at least one release controlling polymer and at least one pharmaceutically acceptable excipient are homogeneously intermixed to form a matrix. Hydrophilic and hydrophobic polymers listed above may be used to prepare these mazindol-containing matrices. These matrices may be presented in the form of matrix tablets, matrix multiparticulates, or in a form of a layer coated onto a substrate.

Matrix tablet formulations are capable of providing a single drug release profile or multiple drug release profiles. Matrix tablet technologies that are capable of providing multiple release profiles include multiple layer tablets (e.g., bilayer or tri-layer tablets), tablet within a tablet technology, encapsulated mini-tablets or a tablet of compressed modified release pellets.

B) Drug-layered systems that comprise an inert core and at least one drug-containing layer coated onto this core. The drug containing layer(s) may be further coated with a layer of a release controlling polymer selected from those listed above. If the drug-containing layer of the drug-layered system does not contain any release-controlling polymers and is of an immediate release, then the release controlling coating is necessary for achieving the modified profiles of the current invention. In the cases when drug-containing layer is an extended-release matrix layer described above, the release controlling coating is optional and allows for additional modification of the release profile.

For example, it may be used to modulate the release (slow initially, faster later; or fast initially, slower later), or to provide a delay in the release. In particular, non-pH-dependent polymers that can be used for coating multiparticulates or tablets (matrix or immediate release) include: cellulose esters, cellulose acetate, cellulose acetate butyrate, ethylcellulose, Eudragit^{®} RS and Eudragit^{®} RL poly (ethyl acrylate-co-methyl methacrylate-cotrimethylammonioethyl methacrylate chloride), Eudragit^{®} NE30D or Eudragit NM30D poly(ethyl acrylate-co-methyl methacrylate), ethyl acrylate methyl methacrylate copolymer, polyvinyl acetate,

In addition, the following enteric compounds can be used in a coating to provide a delay in the release profile: Eudragit^{®} FS30D (poly (methyl acrylate-co-methyl methacrylate-co-methacrylic acid)), Eudragit^{®} L30D-55 Eudragit^{®} L and Eudragit^{®} S (poly (methacrylic acid-co-methyl methacrylate)), hydroxypropyl methylcellulose acetate succinate, hydroxypropyl methylcellulose phthalate, cellulose acetate phthalate, shellac zein, and combinations thereof.

Without putting any limitations thereon, the formulations of this embodiment may be exemplified by the following variations that provide different modified pharmacokinetic (PK) profiles for mazindol:
- Mixed particles in a capsule, compressed tablet or any other dosage form where IR particles are mixed with DR particles (IR/DR mixed particles). The IR particles provide the initial release of the therapeutic agent followed by release from the DR particles resulting in pulsed PK profiles. (IR/DR mixed population of particles)
- A single population of particles in a capsule, compressed tablet or any other dosage form where the pellet incorporates an IR core coated with DR coat which is further coated with an IR drug layer. The outer IR drug layer provides an immediate release of the therapeutic agent followed by a delayed release from the DR core resulting in pulsed PK profile. (IR/DR single population of particles)
- Mixed particles in a capsule, compressed tablet or any other dosage form where IR particles are mixed with DR coated XR particles (IR/DR-XR). The IR particles provide the initial release of the therapeutic agent followed by delayed and extended release from the DR coated XR particles. (IR/DR-XR mixed population of particles)
- A single population of particles in a capsule, compressed tablet or any other dosage form where the pellet incorporates an IR core coated with XR coat, which is coated with DR coat that is subsequently drug layered. The outer drug layer provides the initial release of the therapeutic agent followed by delayed and extended release from the remainder of the pellet. (IR/DR-XR single population of particles)
- Mixed particles in a capsule, compressed tablet or any other dosage form where a fast XR pellet is mixed with a DR pellet. The fast XR provides the initial release of the therapeutic agent followed by release from the DR particles. (XR-f/DR mixed population of particles)
- A single population of particles in a capsule, compressed tablet or any other dosage form where the pellet incorporates IR core coated with a DR coat which is then coated with drug layer that is subsequently coated with an XR coat to produce a fast XR layer. The fast XR outer layer provides the initial release of the therapeutic agent followed by delayed release from the DR core. (XR-f/DR single population of particles)
- A DR tablet coated with an IR drug layer
- One or more than one DR tablets are mixed with one or more than one IR tablets in a capsule
- XR tablet coated with a DR coat, then coated with an IR drug layer
- A bi-layer tablet with one layer containing the drug in XR form and a 2^{nd} layer containing the drug in an IR form
- A bi-layer tablet with one layer containing the drug in XR form and a 2^{nd} layer containing the drug in an DR form
- A DR coated matrix tablet providing an DR/XR profile.

To optimize stability of mazindol in a matrix system, the preferred methods for formulation and processing would be dry methods such as direct compression of a dry powder blend, compression of a roller compacted granulation, compression of a holt melt granulation or a hot melt extrudate. The compressible intermediates (i.e., the dry powder blend, roller compacted granulation, hot melt granulation etc.) can be formulated to be rate controlling in nature (i.e., comprise a drug release rate controlling excipient(s)) or be mixed with release rate controlling excipient(s) prior to tablet compression. Additionally, wet granulations can be manufactured, dried and sized for compression into matrix tablets.

Stabilization techniques, such as using acidic pH media, for the drug substance would be required unless non-aqueous media are employed in the wet granulation process. Additionally, in accordance with the nature of this invention, low moisture content excipients and excipients that by their chemical nature create an acidic environment in the matrix are preferably used. The acidic environment promoted by these excipients can also act to promote the solubility of the drug substance which can be desired in a modified release matrix system formulated to deliver drug in the less acidic regions of the gastrointestinal tract. Stabilization is also achieved by coating drug layered substrates with coating polymers dissolved or dispersed in acidic solution.

Processes useful for producing drug-layered systems include solution or dry powder drug layering onto inert substrates (e.g. sugar or microcrystalline cellulose spheres), spray drying and lyophilization. As mentioned above, due to the chemical instability of mazindol the preferred methods for drug layered systems would be the dry methods (i.e., dry powder drug layering and methods that can process with non-aqueous media, such as spray drying. If the method is to include an aqueous solution in the process (e.g., drug layering), stabilization techniques such as using acidic pH aqueous media may be employed.

Additionally, it is preferred to use low moisture content excipients and excipients that by their chemical nature create an acidic environment. The present inventors have found that excipients with a combination of these properties might provide a synergistic stabilization effect. The acidic environment promoted by these excipients can also act to promote the solubility of the drug substance which can be desired in a modified release drug-layered system formulated to deliver drug in the less acidic regions of the gastrointestinal tract.

C) The osmotic release systems. In a further embodiment, this invention provides an extended release mazindol preparation in the form of an osmotic tablet, wherein the drug release rate is determined by the rate of water permeation into the tablet core through a semi-permeable membrane coating.

For stability of mazindol in an osmotic tablet formulation, the preferred methods for core tablet formulation and processing would be dry methods such as direct compression of a dry powder blend, compression of a roller compacted granulation, compression of a holt melt granulation or a hot melt extrudate. Additionally, fluid bed granulation processes or a high or low shear granulation method can be used when stabilization techniques for the drug substance are employed, such as using acidic pH granulation media or non-aqueous granulation media. It is preferred to use low moisture content excipients and excipients that by their chemical nature create an acidic environment in the core tablet of the osmotic dosage form. The acidic environment promoted by these excipients can also act to promote the solubility of the drug substance which can be a desired attribute when the osmotic tablet formulation is to deliver drug in the less acidic regions of the gastrointestinal tract.

For the preparation of the osmotic tablet, mazindol is mixed with osmotic agent(s), tabletting aides such as diluents and lubricants, and other commonly used excipients. The mixture is tabletted either by direct compression or granulation followed by compression. Tablets are then coated with a semi-permeable rate-controlling membrane.

The semipermeable rate-controlling membrane, which surrounds the drug-containing core, comprises a water insoluble, pharmaceutically acceptable polymer. Suitable water insoluble polymers include, for example, cellulose esters, cellulose ethers and cellulose ester ethers. Non-limiting examples of such polymers include cellulose acylate, cellulose ethyl ether, cellulose diacylate, cellulose triacylate, cellulose acetate, cellulose diacetate, cellulose triacetate, mono-, di- and tricellulose alkyls, mono-, di- and tricellulose aroyls, and combinations thereof.

The semi-permeable rate controlling membrane is applied on the tablets using standard coating techniques such as spraying, dipping, casting, coating solvent evaporation, molding or compression coating. An orifice is drilled on the tablet coat using laser tablet drilling system or other mechanical means to allow the release of drug from the core. The osmotic agents used for the practice of the current invention are well known in the art and include non-swellable compounds represented by, but not limited to, polyols; carbohydrates including monosaccharides, oligosaccharides, polysaccharides and sugar alcohols; salts; acids and hydrophilic polymers.

For example, osmotic agents may be selected from mannitol, maltrin, xylitol, maltitol, lactitol, isomalt, sorbitol, arabitol, erythritol, ribitol, insositol, lactose, glucose, sucrose, raffinose, fructose, dextran, glycine, urea, citric acid, tartaric acid, sodium chloride, potassium chloride, magnesium chloride, disodium hydrogen phosphate, sodium phosphate, potassium phosphate, sodium sulfate, lithium sulfate, magnesium sulfate, magnesium succinate, polyethylene glycol, maltodextrin, cyclodextrins and derivatives, non-swelling block polymers of PEO and PPO, polyols, polyethylene glycols, cellulose ethers, and combinations thereof. Osmotic agents that are acidic by nature may have multiple functions in the formulations of the present invention acting simultaneously as stabilizers. Alternatively, they may provide synergistic action with additional stabilizers.

Osmotic tablets can be formulated as a single or as a multiple layer core. In one embodiment, the osmotic tablet comprises a bilayer core, wherein one layer comprises agents to modulate drug release, such as a solubilizer, that are released in an extended manner, and the second layer comprises the drug and potentially other agents to modulate drug release.

An overcoat of drug can be applied to the tablet following functional coating to provide an immediate release component to the dosage form. Alternatively, the osmotic tablet may be coated with an enteric polymer on top of the semipermeable membrane providing a DR/XR profile.

The embodiments listed above are just non-limiting examples of the modified release stable formulations of mazindol resulting in a product that maintains therapeutic level of the drug in the body from 6 to 24 hrs.

The amount of the drug in a dosage form of the formulations of the instant invention depends on the indication and exact nature of the drug. For mazindol, a daily dose comprises from 0.1mg to 20mg of the drug, preferably from 0.5mg to 10mg. Prodrugs of mazindol that are also within the scope of the instant invention may be delivered in daily doses of from 0.1 mg to 200mg of the active ingredient. For the hydrolysis product of mazindol (HP), the daily dose can vary from 0.1mg to 200mg. For the prodrug of HP, the daily dose can vary from 0.1mg to 400mg.

Mazindol used in the practice of the current invention may be in the form of a single R enantiomer, or in the form of a single S enantiomer, or in the form of a racemic mixture, or in the form of a non-racemic mixture of enantiomers with various amounts of R and S enantiomers. In one embodiment, the amount of an R enantiomer in the mixture is from 0% to 90% by weight of the active pharmaceutical agent. In another embodiment, the amount of R enantiomer is from 0% to 75% by weight of the active pharmaceutical agent. In a further embodiment, it is from 0% to 50%. In a yet further embodiment, it is from 0% to 25%, by weight of the active pharmaceutical agent.

Techniques for enantiomer separation are known to those skilled in the art and include chromatographic techniques using enantio-selective stationary phase, capillary electrophoresis, and liquid-liquid extraction techniques. A particular enantiomer can also be produced directly from the synthetic reaction for the manufacture of mazindol.

In one embodiment of the invention, an R enantiomer of mazindol is used for the treatment of CNS disorders including but not limited to ADHD.

In another embodiment of the invention, an S enantiomer of mazindol is used for the treatment of CNS disorders including but not limited to ADHD.

In the further embodiment of the invention, the use of a mixture of R and S enantiomers in various ratios in the treatment of CNS disorders, including but not limited to ADHD.

The hydrolysis product, 2-(2-Aminoethyl)-3-(4-chlorophenyl)-3-hydroxy-2,3-dihydroxy-1H-isoindol-1-one, may be included into the inventive formulations of mazindol in the amount of from 0% to 100% by weight of the total load of the active pharmaceutical agent. In one embodiment, it is included in the amount of from 0% to 50% by weight of the active pharmaceutical agent. In another embodiment, it is included in the amount of from 0% to 25% by weight of the active pharmaceutical agent.

It was unexpectedly discovered that formulations comprising the hydrolysis product of mazindol may be stabilized and delivered in the same manner and used for the same indications as the inventive formulations comprising non- hydrolyzed mazindol. Thus, current invention also provides for formulations comprising from 0.1mg to 200mg of the hydrolysis product of mazindol (HP) as an active substance. All inventive embodiments disclosed herein for mazindol are fully applicable for the formulations comprising HP or combinations of mazindol with HP. Further, formulations comprising prodrugs that convert into HP in the mammalian body are also within the scope of the instant invention. Such formulations may comprise from 0.1 mg to 400 mg of the prodrug.

The hydrolysis product of mazindol may be used in the form of a pure R enantiomer, or in the form of a pure S enantiomer, or in the form of a mixture of R and S enantiomers in various ratios.

In one additional embodiment, formulations of mazindol as disclosed above may comprise molindone as an additional pharmaceutical ingredient. This embodiment is especially beneficial for the treatment of a subpopulation of patients exhibiting impulsive aggression, aggression, or conduct disorder in the setting of ADHD.

The invention is further illustrated by, though in no way limited to, the following examples.

### EXAMPLES

### Example 1

### Mazindol Immediate Release Pellets with Opadry Overcoat

The composition of Mazindol Immediate Release (IR) pellets is provided in Table 1. IR pellets were manufactured by coating 30/35-mesh sugar spheres with mazindol from a drug layering dispersion consisting of mazindol, hydroxypropylmethylcellulose (Methocel E5PLV, a binder), and talc (an anti-tacking agent) in 0.1N HCl. The drug layering dispersion is prepared by dissolving the drug and Methocel E5PLV in 0.1N HCl followed by dispersing talc in the drug-Methocel E5PLV solution. The resulting dispersion was stirred throughout the drug layering process. Drug layering was carried out in Glatt's GPCG-1 fluid bed coater with the following critical processing parameters: inlet air temperature: 50-60°C, product temperature: 35-45°C, spray rate: 5-10g/min, and atomization air: 1.5 bar. The drug layered pellets were overcoated with Opadry II White in a GPCG-1 fluid bed coater. The total amount of water in the manufactured pellets was less than 5% by weight of the formulation.

Dissolution testing was performed on the pellets using USP Apparatus II at 50RPM and a dissolution medium of 0.1N HCl, pH 1.1

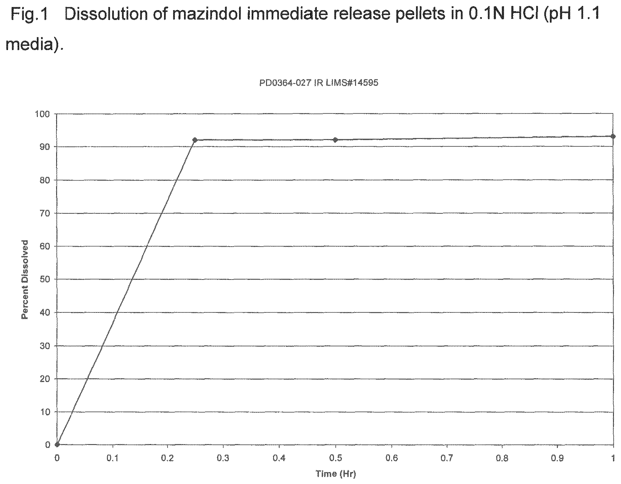

Figure 1 shows the dissolution profile for IR pellets.

| Table 1. IR pellet composition (PD0364-027) | | |
|---|---|---|
| Ingredients | Amount (g) | Amount (%w/w) |
| Mazindol | 120 | 1.50 |
| Sugar Spheres | 7560 | 94.50 |
| Talc | 60 | 0.75 |
| Methocel E5PLV* | 60 | 0.75 |
| Opadry II White* | 200 | 2.50 |
| Total | 8000g | 100.00% |
| | | |

| Drug Layering Dispersion | | |
|---|---|---|
| Mazindol | 120 | 2.50 |
| Talc | 60 | 1.25 |
| Methocel E5PLV | 60 | 1.25 |
| Water (0.1N HCl) | 4560 | 95.00 |
| Total | 4800g | 100.00% |
| | | |

| Opadry Overcoat | | |
|---|---|---|
| Opadry II White | 200 | 10.00 |
| Water | 1800 | 90.00 |
| Total | 2000g | 100.00% |

| | | |
|---|---|---|
| *Methocel E5PLV is a tradename for HPMC; Opadry II White is a PVA-based coating system | | |

### Example 2

### Mazindol Delayed Release Pellets with Opadry Overcoat

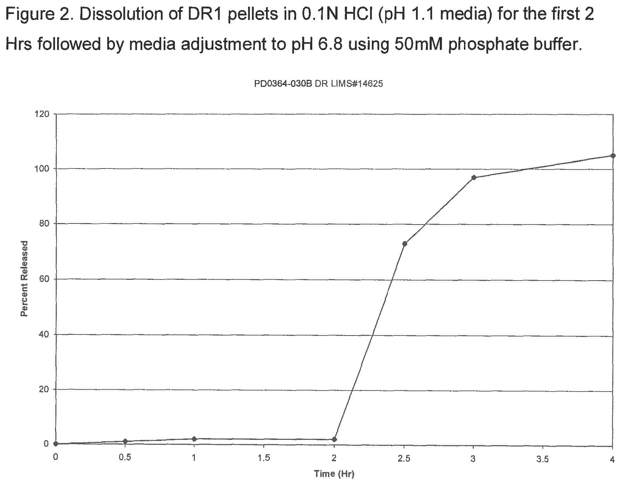

IR Pellets from example 1 were coated with Eudragit^{®}L30D-55 from a coating dispersion consisting of Eudragit L30D-55, triethylcitrate (a plasticizer), talc (anti-tacking agent), and water using Glatt's GPCG-1 fluid bed coater. Figure 2 shows the dissolution profile for the DR1 pellets using USP Apparatus II at 50RPM. The total amount of water in the manufactured pellets was less than 5% by weight of the formulation. The composition of DR1 pellets is provided in Table 2.

| Table 2 Delayed Release (DR1) pellet composition | |
|---|---|
| Ingredients | Amount (%w/w) |
| Immediate release pellets | 57.5 |
| Delayed Release (DR1) coating | 40.0 |
| Opadry II White seal coating | 2.5 |
| Total | 100.0% |

### Example 3

### Encapsulation of IR and DR1 pellets

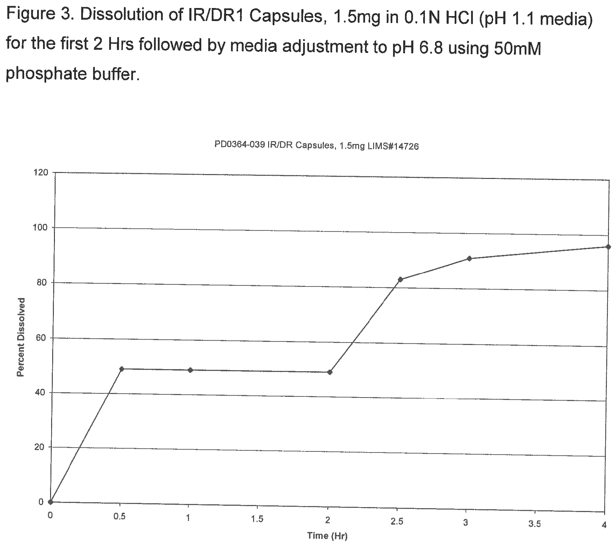

IR and DR1 pellets were encapsulated in size 3 capsules to provide 0.75mg mazindol from the IR pellets and 0.75mg mazindol from DR1 pellets. Figure 3 shows the dissolution profile for IR/DR1 Capsules, 1.5mg, using USP Apparatus II at 50RPM and media of 0.1N HCl (pH 1.1) for the first 2 Hrs followed by media adjustment to pH 6.8 using 50mM phosphate buffer.

### Example 4

### IR pellets with up to 10% w/w Opadry II White overcoat

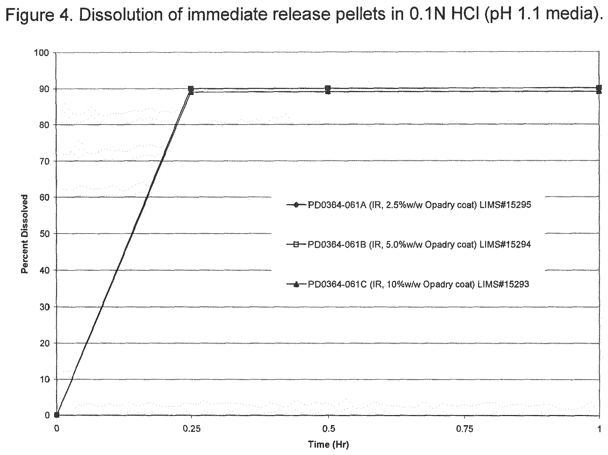

Table 3 provides the composition of IR pellets with varying amount of Opadry coating. Manufacturing followed the same method as in example 1. Figure 4 shows the dissolution profiles of the immediate release pellets using USP Apparatus II at 50RPM and 0.1N HCl (pH 1.1) dissolution media.

| Table 3. IR pellet compositions | | |
|---|---|---|
| Ingredients | Amount (g) | Amount (%w/w) |
| Mazindol | 34.6 | 1.50 |
| Sugar Spheres | 2000.0 | 86.75 |
| Talc (after drug layering) | 23.1 | 1.00 |
| Methocel E5PLV | 17.3 | 0.75 |
| | | 2.50 (A) |
| | | 5.00 (B) |
| Opadry Overcoat | 230.6 | 10.00 (C) |
| Total | 2305.5g | 100.00% |
| | | |

| Drug Layering Dispersion | | |
|---|---|---|
| Mazindol | 34.6 | 2.50 |
| Methocel E5PLV | 17.3 | 1.25% |
| Water (0.1N HCl) | 1331.4 | 96.25% |
| Total | 1383.3g | 100.00% |

| Opadry Overcoat | | |
|---|---|---|
| Opadry II White | 111.1 | 10 |
| Water (0.1N HCl) | 1000.0 | 90 |
| Total | 1111.1g | 100% |

### Example 5

### Drug layering and Aquarius Moisture Guard (AMG) seal coating to 10%

Table 4 provides the composition of IR pellets with varying amount of AMG coating. Drug layering followed the same manufacturing processes as in example 1. Drug-layered immediate release pellets were seal coated with Aquarius Moisture Guard (AMG). AMG is a natural wax-containing pre-formulated powder supplied by Ashland Aqualon (Wilmington, DE). AMG was dispersed in 0.1N HCl to obtain a 20% solids dispersion. The dispersion was mixed for at least 1 Hr prior to coating. Mixing continued throughout the coating process to prevent settling of the AMG. AMG seal coating was carried out in Glatt's GPCG-1 fluid bed coater with the following critical processing parameters: inlet air temperature: 50-60°C, product temperature: 40-45°C, spray rate: 5-10g/min, and atomization air: 1.5bar. The total amount of water in the manufactured pellets was less than 5% by weight of the formulation.

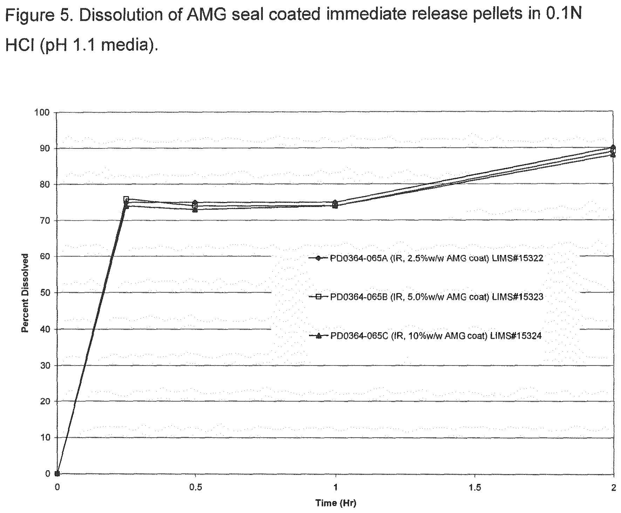

Figure 5 shows the dissolution profiles of the immediate release pellets using USP Apparatus II at 50RPM and 0.1N HCl (pH 1.1) dissolution media.

| Table 4. IR pellet (AMG coated) compositions | | |
|---|---|---|
| Ingredients | Amount (g) | Amount (%w/w) |
| Mazindol | 17.3 | 1.50 |
| Sugar Spheres | 1000.0 | 86.75 |
| Talc (after drug layering) | 11.5 | 1.00 |
| Methocel E5PLV | 8.6 | 0.75 |
| AMG Seal coat | 115.3 | 2.5 (A), 5.0 (B), 10.00 (C) |
| Total | 1152.8g | 100.00% |
| | | |

| AMG Dispersion | | |
|---|---|---|
| AMG | 111.1 | 20.00 |
| Water (0.1N HCl) | 444.4 | 80.00 |
| Total | 555.6g | 100.00% |

### Example 6

### Mazindol Immediate Release Pellets containing tartaric acid (TA) and with Opadry II White Overcoat

The composition of Immediate Release (IR) pellets containing tartaric acid is provided in Table 5. IR (with TA) pellets were manufactured by coating 30/35-mesh sugar spheres with mazindol from a drug layering solution consisting of mazindol, hydroxypropylmethylcellulose (Methocel E5PLV, a binder), and tartaric acid (an acidifying agent) in water. The drug layering dispersion is prepared by dissolving the tartaric acid, dissolving mazindol, and dissolving Methocel E5PLV in water. Drug layering was carried out in Glatt's GPCG-1 fluid bed coater with the following critical processing parameters: inlet air temperature: 50-60°C, product temperature: 35-45°C, spray rate: 5-10g/min, and atomization air: 1.5bar. The drug layered pellets were overcoated with Opadry II White in a GPCG-1 fluid bed coater. The total amount of water in the manufactured pellets was less than 5% by weight of the formulation.

| Table 5. IR pellet compositions | | |
|---|---|---|
| Ingredients | Amount (g) | Amount (%w/w) |
| Mazindol | 17.7 | 1.50 |
| Sugar Spheres | 1000.0 | 84.95 |
| Talc (after drug layering) | 11.8 | 1.00 |
| Methocel E5PLV | 8.8 | 0.75 |
| Tartaric Acid | 21.2 | 1.80 |
| Overcoat | 117.7 | 10.00 |
| Total | 1177.2g | 100.00% |
| | | |

| Drug Layering Dispersion | | |
|---|---|---|
| Mazindol | 17.7 | 2.50 |
| Tartaric Acid | 21.2 | 3.00 |
| Methocel E5PLV | 8.8 | 1.25 |
| Water | 658.6 | 93.25 |
| Total | 706.3g | 100.00% |
| | | |

| Opadry Overcoat | | |
|---|---|---|
| Opadry II White | 117.7 | 20.00 |
| Water | 470.9 | 80.00 |
| Total | 588.6g | 100.00% |

### Example 7

### Mazindol Immediate Release (IR) Tablets

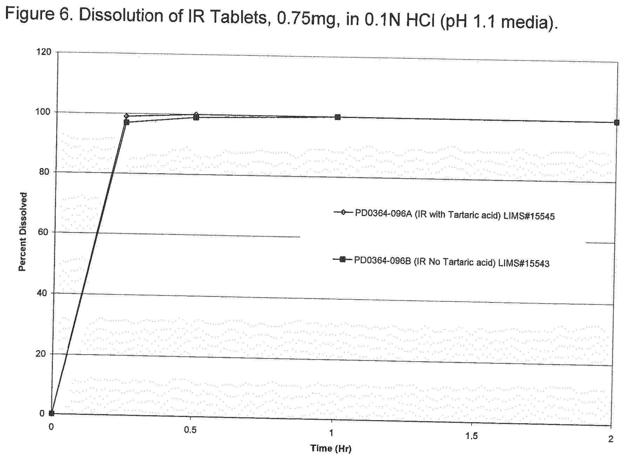

Mazindol IR Tablets were manufactured by direct compression on a Riva Piccola tablet press (SMI, Lebanon, NJ). Table 6 provides the composition of two batches of IR tablets. The batch size for both batches was 500g. Figure 6 shows the dissolution profiles for batches PD0364-096A and PD0364-096B. Dissolution test was performed using USP Apparatus II at 50RPM using 0.1N HCl dissolution media.

| Table 6. Composition of Mazindol IR Tablets, 0.75mg | | |
|---|---|---|
| Ingredients | Amount (%w/w) | Amount (%w/w) |
| Mazindol | 0.8 | 0.8 |
| Prosolv SMCC 90* | 92.2 | 97.2 |
| PVP K25 | 1.0 | 1.0% |
| Magnesium Stearate | 1.0 | 1.0 |
| Tartaric Acid | 5.0 | - |
| Total | 100.0% | 100.0% |

| | | |
|---|---|---|
| * Prosolv SMCC 90- microcrystalline cellulose/colloidal SiO2 | | |

### Example 8

### Mazindol Immediate Release (IR) Tablets

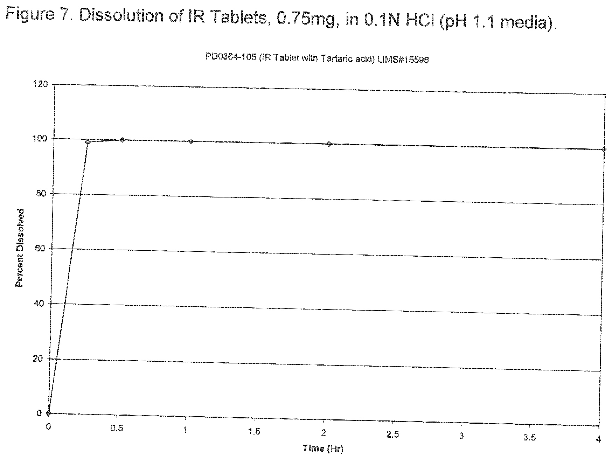

The formulation of this Example is a repeat of the batch in Example 7 with tartaric acid. The batch size was 1kg. Table 7 provides its composition. Figure 7 shows the dissolution profile. Dissolution test was performed using USP Apparatus II at 50RPM using 0.1N HCl dissolution media.

| Table 7. Composition of IR Tablets, 0.75mg | |
|---|---|
| Ingredients | Amount (%w/w) |
| Mazindol | 0.8 |
| Prosolv SMCC 90 | 92.2 |
| PVP K25 | 1.0 |
| Magnesium Stearate | 1.0 |
| Tartaric Acid | 5.0 |
| Total | 100.0% |

### Example 9

### Mazindol Delayed Release (DR1) Tablets

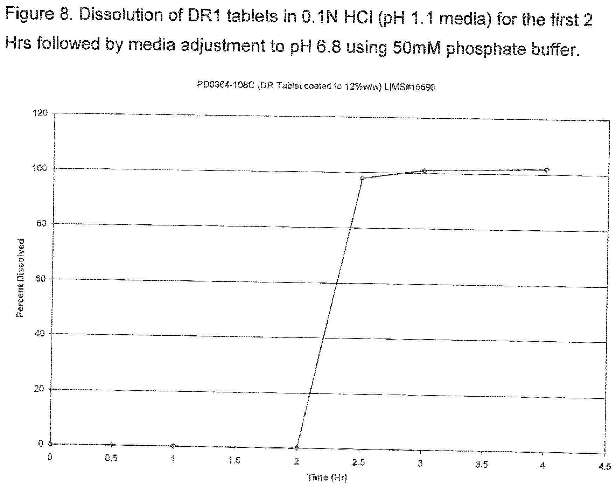

Mazindol IR Tablets of Example 8 were coated with Eudragit^{®}L30D-55 from a coating dispersion consisting of Eudragit L30D-55, triethylcitrate (a plasticizer), talc (anti-tacking agent), and water using Vector's LDCS-III lab coater. Table 8 provides the composition of the DR1 Tablets. Figure 8 shows the dissolution profile for the DR1 tablets using USP Apparatus II at 50RPM. The total amount of water in the manufactured tablets was less than 5% by weight of the formulation.

| Table 8. Composition of Mazindol DR1 Tablets, 0.75mg | |
|---|---|
| Ingredients | Amount (%w/w) |
| IR Tablets (PD0364-105) | 86.0 |
| DR1 Coating | 12.0 |
| Opadry Overcoat | 2.0 |
| Total | 100.0% |

### Example 10

### Mazindol IR Tablets containing anhydrous lactose

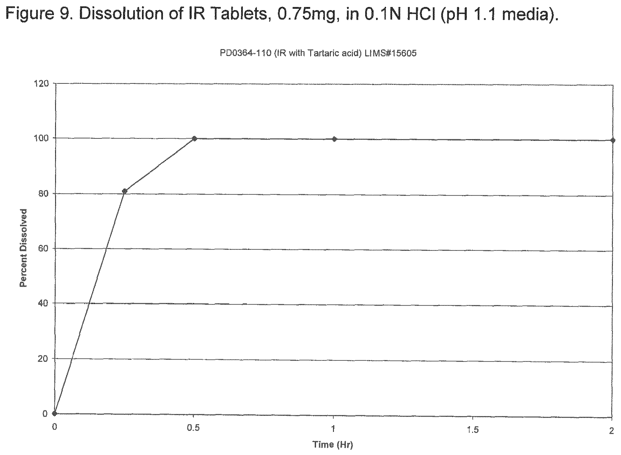

Mazindol IR Tablets containing anhydrous lactose (SuperTab^{®} AN21, DMV-Fonterra) were manufactured by direct compression on a Riva Piccola tablet press (SMI, Lebanon, NJ). Table 9 provides the composition PD0364-110. Figure 9 shows the dissolution profiles PD0364-110. Dissolution test was performed using USP Apparatus II at 50RPM using 0.1N HCl dissolution media. The total amount of water in the manufactured tablets was less than 2% by weight of the formulation.

| Table 9. Composition of Mazindol IR Tablets, 0.75mg | |
|---|---|
| Ingredients | **Amount** (%w/w) |
| Mazindol | 0.8 |
| SuperTab Lactose | 92.2 |
| PVP K25 | 1.0 |
| Magnesium Stearate | 1.0 |
| Tartaric Acid | 5.0 |
| Total | 100.0% |

### Example 11

### Aquarius^{®} Moisture Guard (AMG) seal coated Mazindol IR Tablets

Table 10 provides the composition of AMG seal coated Mazindol IR tablets. Tablets from IR batch of Example 10 were seal coated with Aquarius Moisture Guard (AMG). AMG was dispersed in water to obtain a 10% solids dispersion. The dispersion was mixed for at least 1 Hr prior to coating. Mixing continued throughout the coating process to prevent settling of the AMG components. AMG seal coating was carried out in Vector's LDCS-III lab coater. The total amount of water in the manufactured tablets was 1.56% by weight of the formulation.

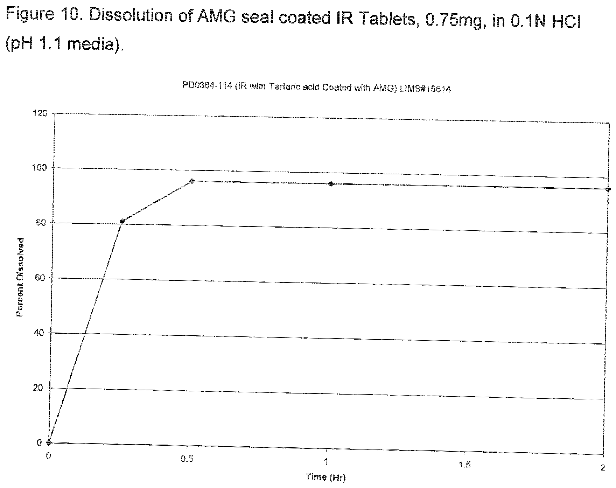

Figure 10 shows the dissolution profiles of the AMG seal coated IR tablets using USP Apparatus II at 50RPM and 0.1N HCl (pH 1.1) dissolution media.

| Table 10. Composition of AMG coated IR Tablets, 0.75mg | |
|---|---|
| Ingredients | Amount (%w/w) |
| SuperTab IR Tablets (PD0364-110) | 95.0 |
| AMG Coat | 5.0 |
| Total | 100.0% |

### Example 12

### Mazindol DR1 Tablets

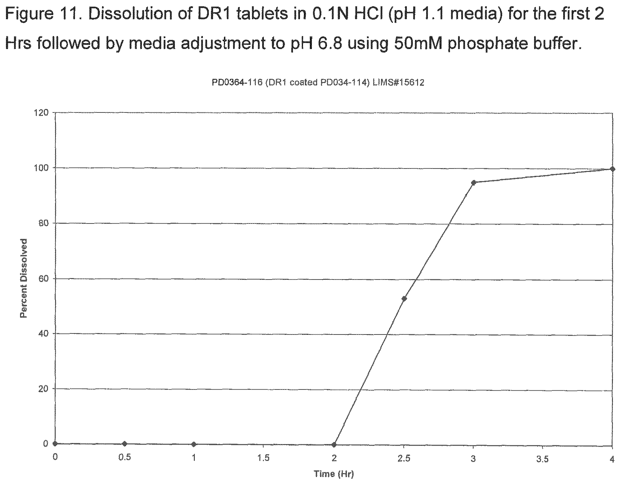

AMG seal coated Mazindol IR Tablets of Example 11 were coated with Eudragit^{®}L30D-55 from a coating dispersion consisting of Eudragit L30D-55, triethylcitrate (a plasticizer), talc (anti-tacking agent), and water using Vector's LDCS-III lab coater. The total amount of water in the manufactured pellets was less than 2% by weight of the formulation. Table 11 provides the composition of Mazindol DR1 Tablets. Figure 11 shows the dissolution profile for Mazindol DR1 tablets using USP Apparatus II at 50RPM.

| Table 11. Composition of Mazindol DR1 Tablets, 0.75mg | |
|---|---|
| Ingredients | Amount (%w/w) |
| IR Tablets (PD0364-114) | 86.0 |
| DR1 Coating | 12.0 |
| Opadry Overcoat | 2.0 |
| Total | 100.0% |

### Example 13

### Mazindol Extended Release (XR1) Tablets

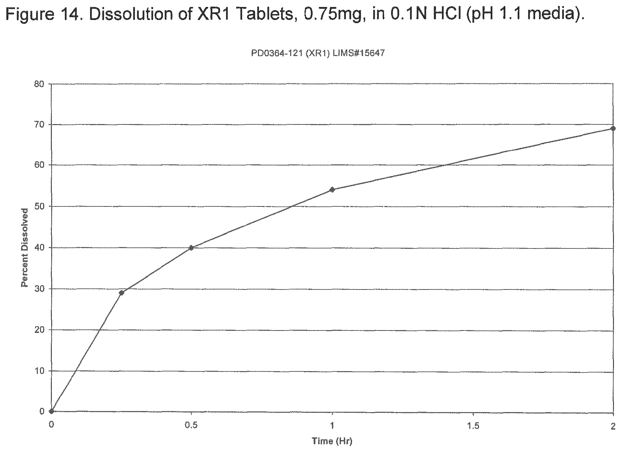

Table 12 provides the composition of Mazindol XR1 Tablets. The XR1 Tablets were manufactured by direct compression on a Riva Piccola tablet press (SMI, Lebanon, NJ). Figure 14 shows the dissolution profiles for the XR1 tablets. Dissolution test was performed using USP Apparatus II at 50RPM using 0.1N HCl dissolution media.

| Table 12. Composition of Mazindol XR1 Tablets, 0.75mg | |
|---|---|
| Ingredients | Amount (%w/w) |
| Mazindol | 0.8 |
| Compritol 888 ATO* | 21.0 |
| Eudragit L100-55* | 10.0 |
| SuperTab Lactose | 62.2 |
| PVP K25 | 1.0 |
| Tartaric Acid | 5.0 |
| Total | 100.0% |

| | |
|---|---|
| * Compritol 888 ATO- glyceryl behenate; Eudragit L100-55- Methacrylic acid ethyl acrylate copolymer | |

### Example 14

### Stability evaluation of Mazindol IR and DR Tablets

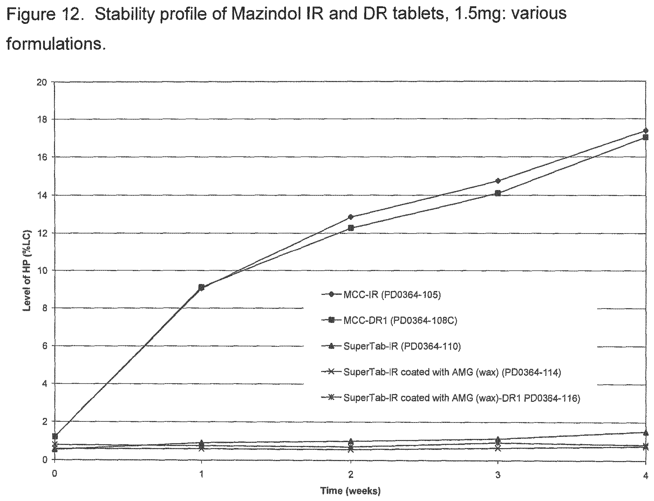

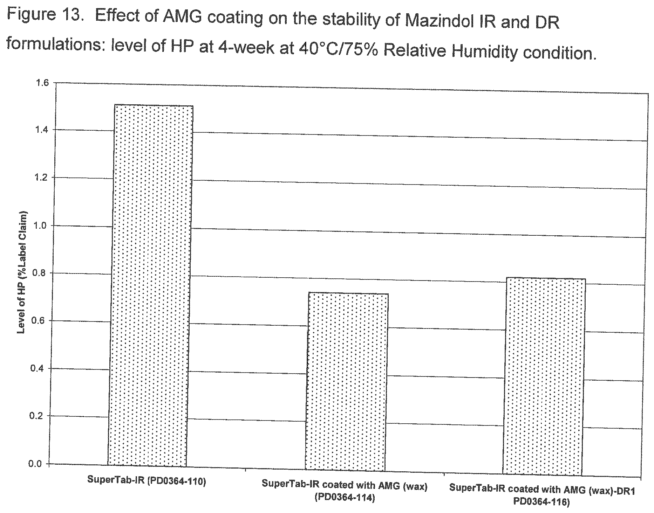

Mazindol IR Tablets of Example 8, DR1 Tablets of Example 9, IR Tablets of Example 10, Moisture Guard film coated IR Tablets of Example 11 and DR1 Tablets of Example 12 were packaged in HDPE bottles and studied for stability at 40°C/75% Relative Humidity conditions. Samples were taken and analyzed weekly for four weeks. Figure 12 shows the stability profiles for the various formulations. The use of anhydrous lactose in the formulation significantly improved the stability of the tablets. Also, the moisture guard coating resulted in improved stability (Fig. 13).

### Example 15

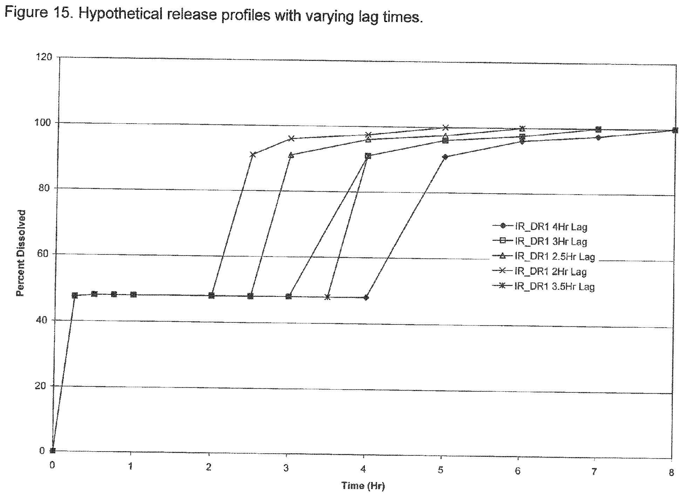

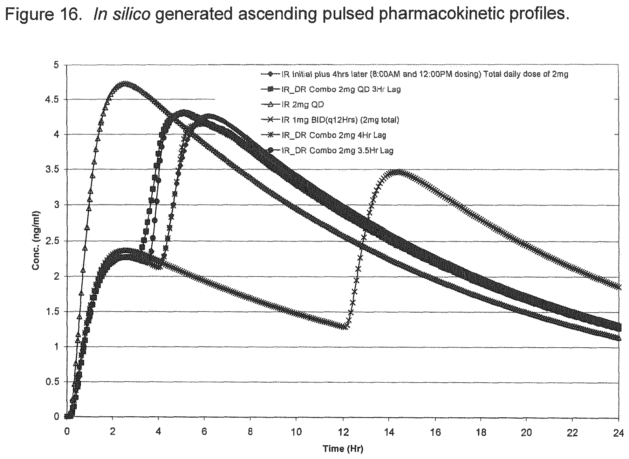

*In silico* modeling was performed to determine various release profiles shown in Figure 15. Figure 16 shows resulting ascending pulsed pharmacokinetic profiles.

### Example 16

Ethanol washing of the mazindol drug substance during manufacturing

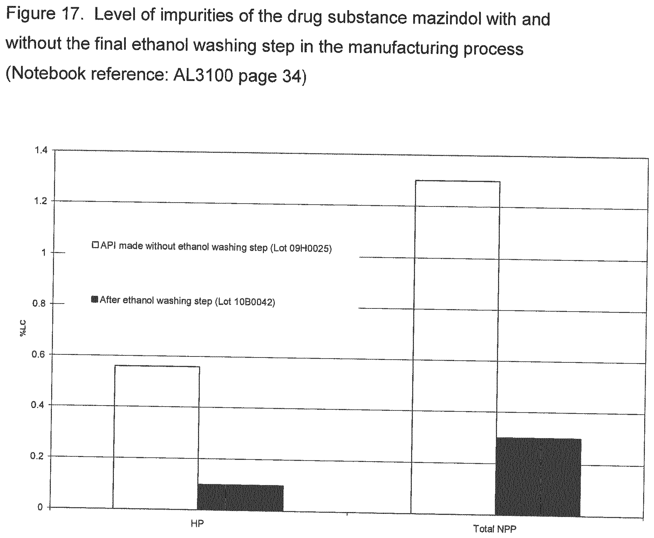

An ethanol washing step was introduced to the manufacturing process of the drug substance, which step resulted in significantly reduced level of impurities as shown in Figure17.

### Example 17

Stability evaluation of Mazindol IR Capsules containing tablets

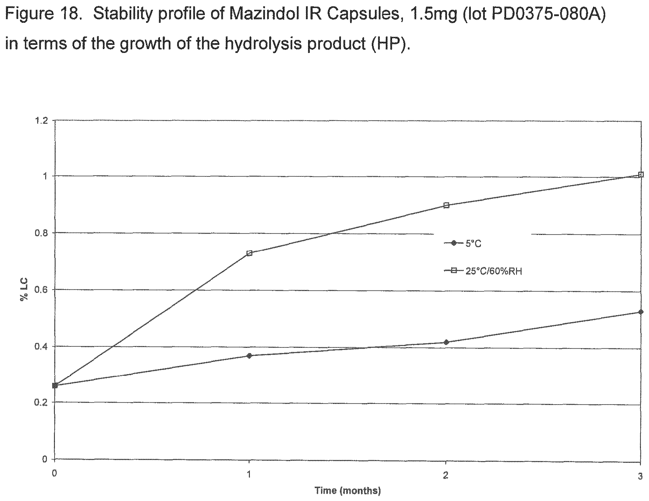

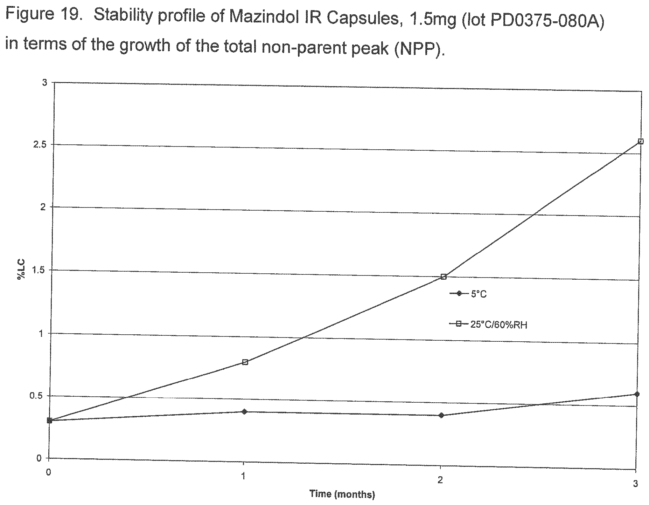

The stability of mazindol IR Capsules, 1.5mg, packaged in blister packs was evaluated at 5°C (2°C - 8°C), and 25°C/60% Relative Humidity conditions. Figures 18 and 19 show the stability profiles of Mazindol IR Capsules, 1.5mg, with respect to the hydrolysis product of mazindol (HP) and total non-parent peaks. The increase in HP as well as other impurities was significantly reduced at the 5°C storage condition.

### Example 18

### Canine study to evaluate the region of absorption of mazindol

### Study design

A total of 6 beagle dogs were assigned to the study (6 males per group x 1 group x 4 phases). All animals were fasted overnight prior to dosing for each phase and through the first 4 hours of blood sample collection (total fasting time not to exceed 24 hours).

### Test Article Administration:

Each animal in Group 1 received a single capsule/tablet dose of the appropriate test article formulation as outlined in the study design in Table 13 below. Each phase was separated by a washout period of 7 days.

**Table 13. Canine study to evaluate the region of absorption of mazindol**

| **Phase/ Group** | **Test Article** | **Number of Males** | **Dose Route** | **Vehicle** | **Target Dose Level (mg/animal)** | **Dose Volume (tablets/capsules/ animal)** | **Matrix Collecte d** |
|---|---|---|---|---|---|---|---|
| 1/1 | Mazindol IR Tablets, 6 mg | 6 | Oral, tablet | NA | 6 | 1 | Blood^{A} |
| 2/1 | Mazindol DR1 Tablets, 6 mg | 6 | Oral, tablet | NA | 6 | 1 | Blood^{A} |
| 3/1 | Mazindol DR2 Tablets, 6 mg | 6 | Oral, tablet | NA | 6 | 1 | Blood^{A} |
| 4/1 | Mazindol CR Capsules, 3 mg | 6 | Oral, capsule | NA | 6 | 2 | Blood^{A} |
| ^{A} Blood samples will be collected predose and at 0.5, 1, 1.5, 2, 2.5, 3, 4, 6, 8, 10, 12, and 24 hours postdose. | | | | | | | |

### Pharmacokinetic Blood Collection

Blood samples were collected from the jugular vein at the time points specified in the study design table above and placed into tubes containing K2 EDTA. All blood samples were placed in an ice block (or wet ice) following collection. The samples were centrifuged within 15 minutes of collection at each interval at approximately 3000 rpm for 15 minutes at approximately 4 deg C. The plasma was separated into two aliquots (primary/retain). Plasma samples were analyzed using LC-MS for both mazindol and the hydrolysis product of mazindol (HP).

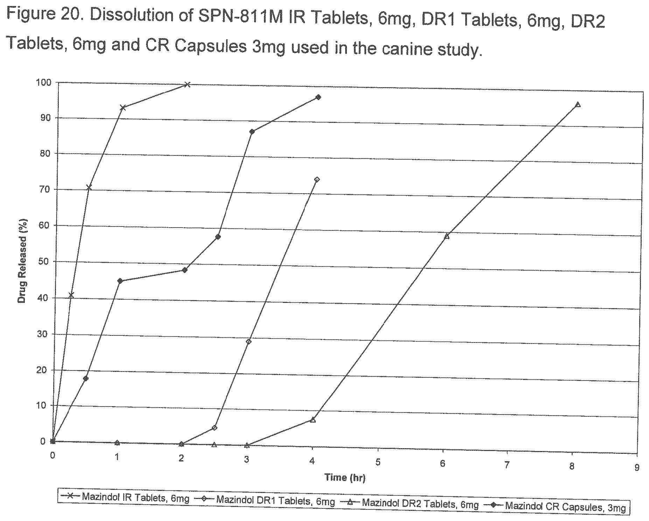

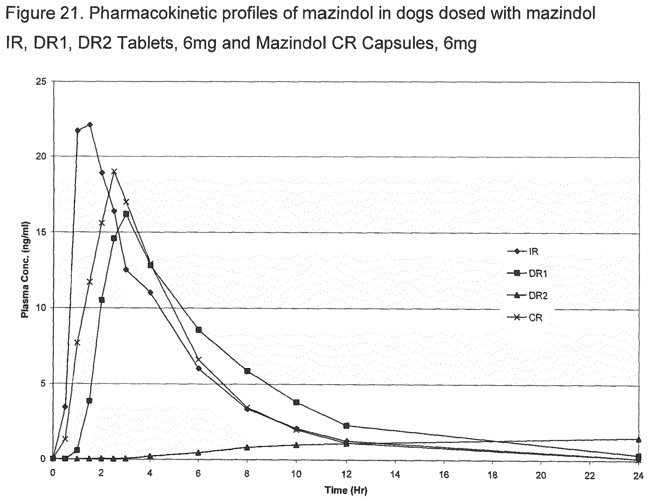

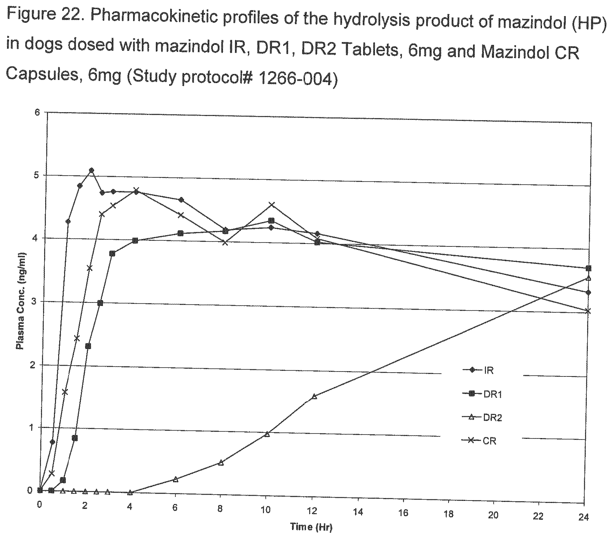

Figure 20 shows the dissolution profiles of the formulations tested. The pharmacokinetic profiles for mazindol and HP are shown in Figure 21 and 22, respectively.

### Example 19

Granulation, tabletting, coating and laser drilling of Mazindol osmotic tablets.

Table 14 provides composition of the granules, cores, and coated tablets.

| Table 14 . Composition of Mazindol granules, uncoated and coated tablets | | | |
|---|---|---|---|
| Formulation | Granules | Uncoated Tablets | Coated Tablets |
| Mazindol | 0.75% | 0.74% | 0.72% |
| Xylitol CM90 | 43.23% | 42.91% | 41.62% |
| Maltrin M150 (wet)* | 1.41% | 1.40% | 1.36% |
| Maltrin M150 (dry)* | 49.57% | 49.20% | 47.72% |
| Tartaric acid | 5.04% | 5.00% | 4.85% |
| Magnesium Stearate | NA | 0.75% | 0.73% |
| Cellulose Acetate | NA | NA | 2.40% |
| Triethyl Citrate | NA | NA | 0.60% |
| Total | 100.00% | 100.00% | 100.00% |

| | | | |
|---|---|---|---|
| * Maltrin M150 is a tradename for maltodextrin | | | |

All excipients are screened through an 18-mesh sieve prior to granulation. Granules are manufactured by top spray granulation in Glatt's fluid bed granulator (GPCG-1 or GPCG-15 (Glatt^{®} Air Techniques Inc., Ramsey, NJ )). Two spray solutions are prepared: Solution 1 containing Maltrin M150 (used as a binder), tartaric acid, and the drug, mazindol. Solution 2 containing Maltrin M150 only. Prescreened excipients are charged into the fluid bed granulator. Spray solution 1 is sprayed first followed by spray solution 2. Granulation process parameters are provided in Table 15

Upon spraying, the granules are dried in the fluid bed while monitoring the moisture level. A moisture level of less than 3% by weight of the formulation is considered acceptable. Dried granules are screened through an 18-mesh sieve.

| Table 15. Granulation processing parameters | | |
|---|---|---|
| | Lab scale | CTM Scale |
| Fluid bed Granulator | GPCG-1 | GPCG-15 |
| Typical batch size (kg) | 2 | 10 |
| Inlet air temperature (°C) | 58-63 | 58-63 |
| Exhaust air temperature (°C) | 28-31 | 28-31 |
| Product temperature (°C) | 31-33 | 31-33 |
| Air volume (m/s for GPCG1 and CFM for GPCG-15) | 4 | 400-500 |
| Spray rate (g/min) | 8-9 | 120-150 |

Screened granules are blended with magnesium stearate in a V-blender run for 3 minutes and tabletted on Stokes Riva Piccola tablet press using a 5/16" round standard concave tooling. Tablet weights, hardness, and thickness are monitored throughout the compression run.

Core tablets are coated with a coating system containing cellulose acetate as a polymer and triethylcitrate as a plasticizer. Coating is performed in a LDCS-III pan coater (Vector Corporation, Marion, IA) to achieve various coating thickness as determined by the level of weight gain on the core tablet.

An orifice is drilled on the coated tablets using Lumonics laser tablet drilling system (Resonetics Inc, Nashua, NH). The laser power and beam diameter are adjusted to achieve various hole sizes.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

## Claims

1. A modified release formulation of mazindol wherein immediate release pellets and delayed release pellets are encapsulated comprising:
a. a first mazindol-containing component which is a delayed release pellet component comprising:
(1) an immediate release core consisting of 1,5% by weight of mazindol as an active pharmaceutical ingredient and 0,75% by weight of hydroxypropyl methylcellulose as a pharmaceutically acceptable low-moisture excipient and 0,75% by weight of talc as an anti-tacking agent coated on 94,5% by weight of sugar spheres;
(2) a PVA-based seal coat surrounding the immediate release core;
(3) Eudragit^{®} L30D-55 as a delayed release coating surrounding the seal coat;
wherein the total water in the manufactured pellets is less than 5 % by weight of the formulation; and
wherein the amount of the immediate release core is 57,5% by weight of the composition of the delayed release component, wherein the amount of the seal coat is 2,5% by weight of the composition of the delayed release component, and wherein the amount of the delayed release coating is 40% by weight of the composition of the delayed release component,
b. a second mazindol-containing component which is an immediate release pellet component comprising:
(1) an immediate release core consisting of 1,5% by weight of mazindol as an active pharmaceutical ingredient and 0,75% by weight of hydroxypropyl methylcellulose as a pharmaceutically acceptable low-moisture excipient and 0,75% by weight of talc as an anti-tacking agent coated on 94,5% by weight of sugar spheres; and
(2) 2,5% by weight of a PVA-based seal coat surrounding the immediate release core, each based on the total weight of the immediate release component;
wherein the total water in the manufactured pellets is less than 5 % by weight of the formulation;
wherein the total amount of water in the formulation is less than 5% by weight of the formulation, and wherein the first mazindol-containing component and the second mazindol-containing component are encapsulated to provide 0,75mg mazindol from the delayed release component and 0,75 mg mazindol from the immediate release component.

2. The formulation of claim 1 for once-a-day administration or twice-a-day administration.

3. Use of an effective amount of a dosage form of anyone of the preceding claims in the manufacture of a pharmaceutical preparation for treating ADHD.

## Patentansprüche

1. Mazindol-Formulierung mit modifizierter Freisetzung, wobei Pellets mit sofortiger Freisetzung und Pellets mit verzögerter Freisetzung eingekapselt sind, umfassend:
a. eine erste Mazindol-haltige Komponente, welche eine Pelletkomponente mit verzögerter Freisetzung ist, umfassend:
(1) einen Kern mit sofortiger Freisetzung, bestehend aus 1,5 Gew.-% Mazindol als einem pharmazeutischen Wirkstoff und 0,75 Gew.-% Hydroxypropylmethylcellulose als einem pharmazeutisch verträglichen, feuchtigkeitsarmen Hilfsstoff sowie 0,75 Gew.-% Talkum als einem Antiklebemittel, beschichtet auf 94,5 Gew.-% Zuckerkügelchen;
(2) eine Versiegelungsschicht auf PVA-Basis, die den Kern mit sofortiger Freisetzung umgibt;
(3) Eudragit^{®} L30D-55 als eine Beschichtung mit verzögerter Freisetzung, die die Versiegelungsschicht umgibt;
wobei der Gesamtwassergehalt in den hergestellten Pellets weniger als 5 Gew.-% der Formulierung beträgt; und
wobei die Menge des Kerns mit sofortiger Freisetzung 57,5 Gew.-% der Zusammensetzung der Komponente mit verzögerter Freisetzung beträgt, wobei die Menge der Versiegelungsschicht 2,5 Gew.-% der Zusammensetzung der Komponente mit verzögerter Freisetzung beträgt, und wobei die Menge der Beschichtung mit verzögerter Freisetzung 40 Gew.-% der Zusammensetzung der Komponente mit verzögerter Freisetzung beträgt,
b. eine zweite Mazindol-haltige Komponente, die eine Pelletkomponente mit sofortiger Freisetzung ist, umfassend:
(1) einen Kern mit sofortiger Freisetzung, bestehend aus 1,5 Gew.-% Mazindol als einem pharmazeutischen Wirkstoff und 0,75 Gew.-% Hydroxypropylmethylcellulose als einem pharmazeutisch verträglichen, feuchtigkeitsarmen Hilfsstoff sowie 0,75 Gew.-% Talkum als einem Antiklebemittel, beschichtet auf 94,5 Gew.-% Zuckerkügelchen; und
(2) 2,5 Gew.-% einer Versiegelungsschicht auf PVA-Basis, die den Kern mit sofortiger Freisetzung umgibt, jeweils basierend auf dem Gesamtgewicht der Komponente mit sofortiger Freisetzung;
wobei der Gesamtwassergehalt in den hergestellten Pellets weniger als 5 Gew.-% der Formulierung beträgt;
wobei die Gesamtwassermenge in der Formulierung weniger als 5 Gew.-% der Formulierung beträgt und wobei die erste Mazindol-haltige Komponente und die zweite Mazindol-haltige Komponente so eingekapselt sind, dass 0,75 mg Mazindol aus der Komponente mit verzögerter Freisetzung und 0,75 mg Mazindol aus der Komponente mit sofortiger Freisetzung bereitstellen.

2. Formulierung nach Anspruch 1 zur einmaligen Verabreichung pro Tag oder zweimaligen Verabreichung pro Tag.

3. Verwendung einer wirksamen Menge einer Darreichungsform gemäß einem der vorstehenden Ansprüche zur Herstellung einer pharmazeutischen Zubereitung zur Behandlung von ADHS.

## Revendications

1. Formulation de mazindol à libération modifiée dans laquelle des pastilles à libération immédiate et des pastilles à libération retardée sont encapsulées, comprenant :
a. un premier composant contenant du mazindol qui est un composant de pastille à libération retardée comprenant :
(1) un noyau à libération immédiate constitué de 1,5 % en poids de mazindol en tant que principe actif pharmaceutique et de 0,75 % en poids d'hydroxypropylméthylcellulose en tant qu'excipient à faible teneur en humidité pharmaceutiquement acceptable et de 0,75 % en poids de talc en tant qu'agent anti-agglomérant enrobé sur 94,5 % en poids de sphères de sucre ;
(2) un revêtement de scellement à base de PVA qui entoure le noyau à libération immédiate ;
(3) Eudragit^{®} L30D-55 en tant que revêtement à libération retardée qui entoure la couche de revêtement de scellage ;
dans laquelle la teneur totale en eau dans les pastilles fabriquées est inférieure à 5 % en poids de la formulation ; et
dans laquelle la quantité du noyau à libération immédiate est de 57,5 % en poids de la composition du composant à libération retardée, dans laquelle la quantité de la couche de revêtement de scellage est de 2,5 % en poids de la composition du composant à libération retardée, et dans laquelle la quantité du revêtement à libération retardée est de 40 % en poids de la composition du composant à libération retardée,
b. un second composant contenant du mazindol qui est un composant de pastille à libération immédiate comprenant :
(1) un noyau à libération immédiate constitué de 1,5 % en poids de mazindol en tant que principe actif pharmaceutique et de 0,75 % en poids d'hydroxypropylméthylcellulose en tant qu'excipient à faible teneur en humidité pharmaceutiquement acceptable et de 0,75 % en poids de talc en tant qu'agent anti-agglomérant enrobé sur 94,5 % en poids de sphères de sucre ; et
(2) 2,5 % en poids d'une couche de revêtement de scellage à base de PVA qui entoure le noyau à libération immédiate, chacun sur la base du poids total du composant à libération immédiate ;
dans laquelle la teneur totale en eau dans les pastilles fabriquées est inférieure à 5 % en poids de la formulation ;
dans laquelle la quantité totale d'eau dans la formulation est inférieure à 5 % en poids de la formulation, et dans laquelle le premier composant contenant du mazindol et le second composant contenant du mazindol sont encapsulés afin d'obtenir 0,75 mg de mazindol à partir du composant à libération retardée et 0,75 mg de mazindol à partir du composant à libération immédiate.

2. Formulation selon la revendication 1 destinée à une administration une fois par jour ou deux fois par jour.

3. Utilisation d'une quantité efficace d'une forme posologique selon l'une quelconque des revendications précédentes dans la fabrication d'une préparation pharmaceutique destinée à traiter un THADA.
